# EUROPEAN PATENT APPLICATION

(11) **EP 4 140 527 A1**
(43) Date of publication of application: **01.03.2023**
(21) Application number: 21793037.9
(22) Date of filing: 19.04.2021
(51) Int. Cl.: A61M 25/02, A61B 1/307, A61M 31/00

(54) **REVERSIBLE RING FOR MALE URETHRAL COMPRESSION**

(30) Priority: 23.04.2020 ES 202030336
(71) Applicant: Fuentes Muries, José Ángel, 03340 Albatera (Alicante) (ES)
(72) Inventor: BERNÁ SERNA, Juan De Dios, 03340 Albatera (Alicante) (ES); BERNÁ MESTRE, Juan De Dios, 03340 Albatera (Alicante) (ES); FUENTES MURIES, José Ángel, 03340 Albatera (Alicante) (ES)
(74) Representative: Sahuquillo Huerta, Jesús
(86) International application number: PCT/ES2021/070255
(87) International publication number: WO 2021/214357

(57) **Abstract**

Disclosed is a reversible ring for male urethral compression, which comprises a semicircular base (1) formed by a case and a cover of same, wherein the base (1) includes a locking tab (2), anti-slip imprints (3) and a flat belt (4) covered by a padded ring (7), a probe-holding clamp (5) being attached thereto, which is connected to the base (1) by means of a rod (6). The reversible ring is designed for carrying out (simply) both conventional retrograde urethrography (RUG) and new imaging techniques: urethrosonography (USG), urethro-computed tomography (urethro-CT) and urethro-magnetic resonance imaging (urethro-MRI), allowing the retrograde instillation of various radiological contrasts into the male urethra and bladder.

## Description

### Object of the invention

The object of the present invention is a reversible ring for male urethral compression, which includes a probe-holding clamp for diagnostic or therapeutic urethral instillations, and which allows to perform (in a simple way) both the conventional technique of retrograde urethrography (UGR), as well as the new imaging techniques: urethrosonography (USG), urethro-Computed Tomography (urethro-CT) and urethro-Magnetic Resonance Imaging (urethro-MRI), by allowing the different radiological contrasts to be instilled retrograde in the male urethra and bladder.

The incorporation of a system for blocking and unblocking compression by means of the padded ring on the penis allows the urethra to be reversibly compressed and decompressed, without the need to use scissors to cut the flange and decompress. In addition, it will allow therapeutic instillations (drugs, chemotherapy, ...) of the urethra and bladder, as the compression of the ring is reversible, and the procedure can be performed intermittently. Finally, the incorporation of the catheter blocking notch in the clamp prevents intra-urethral fluid from leaking out of the catheter when disconnecting it from the infusion system or syringe and allows the patient to move and facilitates intermittent cysto-urethral instillation.

### Background to the invention

Male urethral pathology, especially urethral strictures, is a common problem and a major cause of morbidity and discomfort. Retrograde urethrography (UGR) is the most used diagnostic test to assess urethral abnormalities. The most used technique is the balloon Foley catheter placed in the distal portion of the penile urethra, filling the balloon with saline until it becomes firmly fixed.

However, there are drawbacks to this procedure, as the filling of the catheter balloon and the consequent urethral distention causes discomfort and discomfort to the patient. In addition, there is a risk of injuring the urethral mucosa and causing urethrorrhagia. The balloon catheter technique is also difficult or impossible to perform in patients with distal urethral strictures or in cases with previous surgery of the urethromeatal area, as the balloon cannot be inflated.

Another procedure is known (Clamp Method) to facilitate retrograde urethrography by means of a device registered as patent ES 2 329 534, which describes an improved device to facilitate the performance of male urethrography, characterized by being composed of the following parts or elements: a semi-circular housing and housing cover system, intended to serve as a cradle for supporting the ventral portion of the penis; a clamp rod integrated between the housing and the housing cover and adapted for the immobilization of various probes; a padded ring between the casing and the casing cover allows an adequate coupling between the device and the penis, a flat strap of adjustable length, located between the casing and the casing cover, which ensures its fixation and positioning; this flat strap is provided with parallel, successive and close transversal grooves on one of its sides, in order to form a clamp of adjustable dimension and compression by the interlocking of these transversal grooves; anti-slip treads on the outer faces of the housing and the housing cover to facilitate gripping and handling of the device, the shape of which is perfectly adapted to the fingers of the person handling the device; the housing and housing cover system is fitted with a V-shaped recess to facilitate cutting of the flat belt at the end of the urethrographs, the scissors or cutting element being able to be inserted through this recess to cut the belt.

The clamp method, which is performed with the device described in this report, is a good alternative as it increases patient comfort and relaxation as it is a painless procedure and allows cases with urethro-meatal alterations to be performed using a thin pre-lubricated probe (6Fr) without a balloon (Berná-Mestre, et al., "Urethrography in men: Conventional Technique versus Clamp Method", Radiology, vol. 252, pp. 240-246, 2009).

Retrograde urethrography is the standard imaging study to evaluate male urethral strictures. However, retrograde urethrography has limitations such as underestimation of the length of the urethral stricture and failure to visualize periurethral tissue. To visualize periurethral anatomy and pathology, as well as to make accurate measurements of the stricture and surrounding spongiofibrosis, new imaging methods such as urethrosonography (USG), urethro-CT and urethro-MRI must be used. The clamp method allows these procedures to be performed easily and accurately.

The urethrosonography performed with the present invention and an infusion system with the bottle positioned at 2m, allows the procedure to be performed painlessly and without syringes. In addition, two operators are usually required to perform urethrosonography. Another great advantage of using the device with the infusion system is that only one operator is needed to perform the urethrosonography (Berná-Mestre, et al., "Optimisation of sonourethrography: the clamp method", European Radiology, vol. 28, pp. 1961-1968, 2018).

The device corresponding to the patent above-mentioned patent ES 2 329 534 presents a problem because the external compression of the penis is not reversible and to remove the device it is necessary to cut the strap with scissors, making the decompression irreversible. A solution to this problem provided by the present invention is the built-in strap locking system, which allows reversible and intermittent compression and decompression, without the need to cut the strap.

In addition, as the compression of the ring is reversible and the procedure can be performed intermittently, this allows therapeutic instillations (drugs, chemotherapy, etc.) of the urethra and bladder, which require a longer procedure time than in diagnostic tests, allowing the ischemia produced in the glans penis after compression of the ring to be "paused". In addition, the incorporation of the catheter blocking recess in the clamp prevents intra-urethral fluid from leaking out of the catheter when disconnecting it from the infusion system or syringe and allows the patient to move and facilitates intermittent cysto-urethral instillation. Finally, the modification of the clamp's orifice calibre allows 6 and 10 Fr catheters to be fixed more securely than in the previous device model.

### Description of the invention

The reversible ring for male urethral compression, the subject of the present invention, consists essentially of a semi-circular base incorporating a locking and unlocking tab for the strap inserted in a circular shape between the base and the padded ring.

The flange in free position allows the strap to be pulled freely, reducing the calibre of the padded ring and performing external compression, so that when the flange is changed to the locking position, the strap is immobilized, which allows external compression to be maintained and reversible, since when the flange is changed to free position, the ring recovers its initial calibre and decompresses, and compression and decompression can be exercised again intermittently, when the locking flange is moved.

In addition, a urethral catheter locking recess has been incorporated into the catheter clamp, and the calibre of the two holes has been adjusted so that 10 Fr or 6 Fr urethral catheters can be attached.

Thanks to the present invention, the urethrosonography performed with the present invention will allow the procedure to be carried out minimizing the need for operators (only one operator in the performance of the procedure), and in turn, will allow the infusion system to be located with the bottle placed at a distance of two meters, all of which will result in the possibility of performing the procedure without pain and without syringes, with the consequent benefits for the patient that this will produce.

### Brief description of the figures

The following is a very brief description of a series of drawings which help to better understand the invention, and which relate expressly to an embodiment of the invention which is presented as a non-limiting example of the invention.

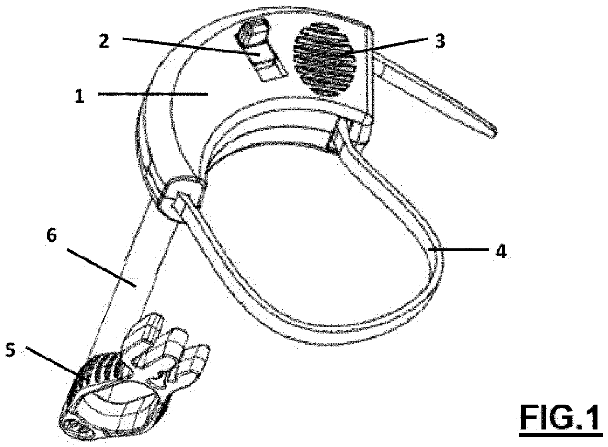

- FIG 1.: It shows a view of the reversible ring for male urethral compression, object of the present invention. Wherein, the semi-circular base (1) incorporates a locking tab (2) and built-in anti-slip treads (3), the flat strap (4) and the probe holder clamp (5) which is attached to the base by means of a rod (6).

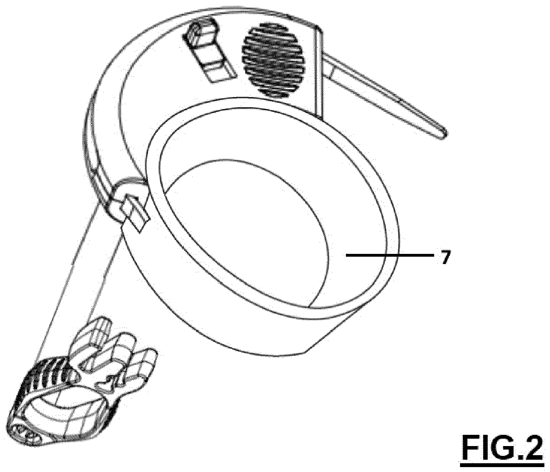

- FIG 2.: It shows a second view of the reversible ring for male urethral compression, where the padded ring (7) prevents the strap from contacting the skin, so that, when the strap is pulled at its free end, the ring's calibre is reduced, and external compression is achieved.

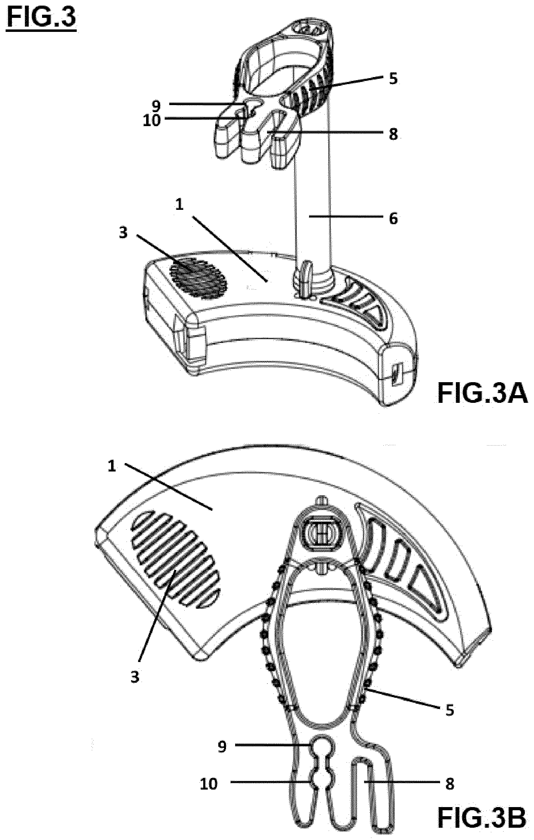

- FIG 3.: It shows a view of the probe holder clamp (5), where view 3A shows a side view, while view 3B shows a front view of the probe holder clamp (5).

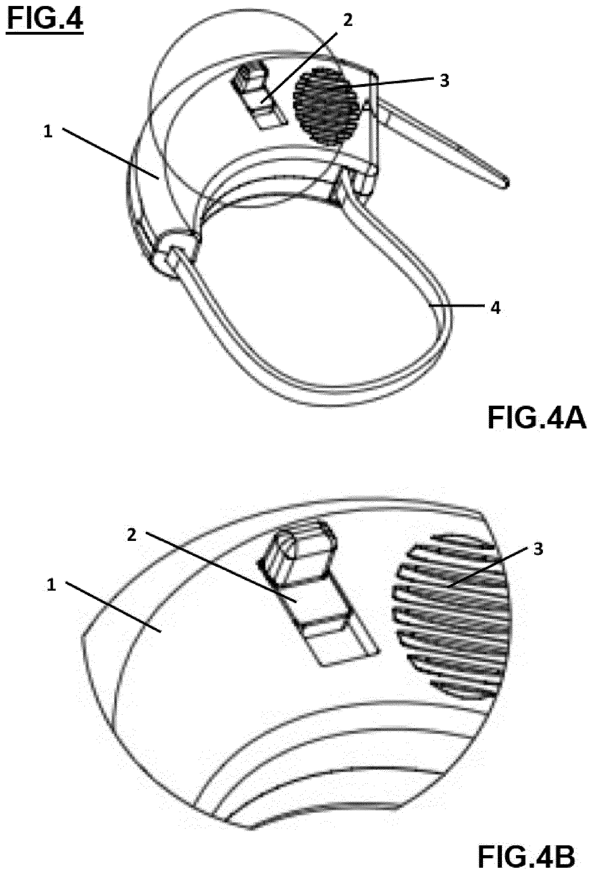

- FIG 4.: It shows a view of the belt locking system of the flat strap (4) where FIG 4A shows a perspective view and FIG. 4B shows an enlarged detail view of the locking tab (2), with the housing cover mounted on the base (1).

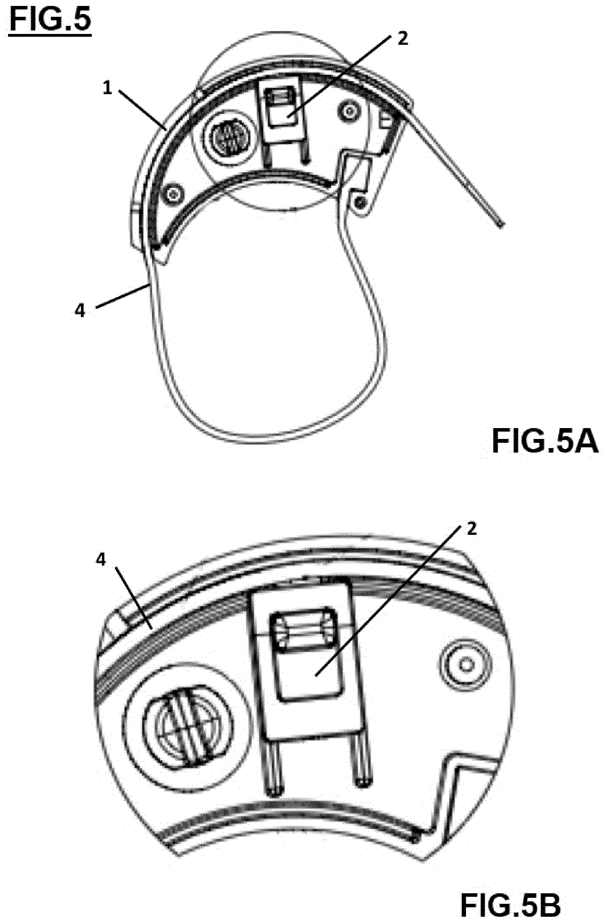

- FIG 5. FIG. 5A: shows a view of the locking position of the strap (4), wherein view 5A shows a perspective view and FIG. 5B shows an enlarged detail view.

### Detailed description of an embodiment of the invention

A preferred embodiment of the invention is shown in the attached figures. More specifically, the reversible ring for male urethral compression, object of the present invention, is characterised in that it comprises a semi-circular base (1) consisting of a housing and the cover thereof; and wherein said base (1) houses a locking tab (2), anti-slip treads (3), and wherein said base (1) houses a flat strap (4) covered by a padded ring (7); and is fitted with a probe holder clamp (5) connected to the base (1) by means of a rod (6).

The locking tab (2), incorporated in the semi-circular base (1), allows it to be moved and clicked to switch reversibly between the locked or free position of the strap (4).

The anti-slip treads (3), located on the outer sides of the semi-circular base (1), prevent the ring from slipping and make it easier to hold and handle.

The probe holder clamp (5) incorporates two flexible side bands which, when tightened, open the clamp (5), allowing the 6 Fr probe to be placed in the internal orifice (9) or the 10 Fr probe in the external orifice (10), so that when the clamp (5) is released, the clamp (5) closes, and the probe is immobilised in the clamp.

In addition, the clamp (5) incorporates a catheter blocking recess (8).

The rod (6) connecting the clamp (5) to the base (1) of the ring will be long enough to leave the glans free, by distancing the compression of the ring in the balanopreputial groove to the clamp (5) where the urethral catheter is fixed.

The padded ring (7) prevents discomfort caused by compression of the penis. The flat strap (4), which is adjustable in length and has parallel and successive transverse grooves on one side, runs inside the ring to form an adjustable and reversible compression mechanism by means of the built-in locking tab (2).

## Claims

1. Reversible ring for male urethral compression, **characterised in that it** comprises a semi-circular base (1) made up of a casing and the cover of the same; and where said base (1) houses a locking tab (2), anti-slip treads (3), and where said base (1) houses a flat strap (4) covered by a padded ring (7); and is fitted with a probe holder clamp (5) connected to the base (1) by means of a rod (6).

2. Reversible ring for male urethral compression according to claim 1, where the probe holder clamp (5) incorporates two flexible side bands which, when tightened, open the clamp (5), allowing the 6 Fr probe to be placed in the internal orifice (9) or the 10 Fr probe in the external orifice (10).

3. Reversible ring for male urethral compression according to any of claims 1 - 2 wherein the clamp (5) incorporates a catheter blocking recess (8).

4. Reversible ring for male urethral compression according to any of the previous claims, where the flat strap (4) is provided with parallel and successive transversal grooves on one of its sides, to configure an adjustable and reversible compression mechanism, by means of the incorporated locking tab (2).
